# EUROPEAN PATENT APPLICATION

(11) **EP 2 730 171 A1**
(43) Date of publication of application: **14.05.2014**
(21) Application number: 12807539.7
(22) Date of filing: 03.07.2012
(51) Int. Cl.: A23F 5/04, A61K 8/97, A61K 36/74, A23L 1/30, A23L 3/3472

(54) **APPLICATION OF PRODUCTS OF COFFEE SILVERSKIN IN ANTI-AGEING COSMETICS AND FUNCTIONAL FOOD**

(30) Priority: 04.07.2011 ES 201131128
(71) Applicant: Consejo Superior De Investigaciones Científicas (CSIC), 28006 Madrid (ES)
(72) Inventor: DEL CASTILLO BILBAO, María Dolores, E-28049 Madrid (ES); IBÁÑEZ EZEQUIEL, María Elena, E-28049 Madrid (ES); AMIGO BENAVENT, Miryam, E-28049 Madrid (ES); HERRERO CALLEJA, Miguel, E-28049 Madrid (ES); PLAZA DEL MORAL, Merichel, E-28049 Madrid (ES); ULLATE ARTIZ, Mónica, E-28049 Madrid (ES)
(74) Representative: Ungria López, Javier
(86) International application number: PCT/ES2012/070490
(87) International publication number: WO 2013/004873

(57) **Abstract**

The invention relates to a product obtained from coffee silverskin, characterised in that it is (a) powdered coffee silverskin or b) coffee silverskin extract. The invention also relates to a method for extracting coffee silverskin, comprising a step of extraction with water, preferably with subcritical water. The invention further relates to the use of the product obtained from coffee silverskin in cosmetics or functional food, especially for preventing physiological ageing processes, as a food preservative for preventing oxidative processes and as a source of caffeine.

## Description

### FIELD OF THE INVENTION

The present invention relates to a method for re-valorisation of a coffee by-product obtained during the roasting process: coffee silverskin. The silverskin can be used directly as a natural source of antioxidants. The antioxidants that make up the silverskin withstand the process of gastrointestinal digestion and as such are bioavailable. A simple extraction process can be used to obtain coffee silverskin extract with antioxidant properties, containing different bioactive compounds such as chlorogenic acid and caffeine, which have cosmetic, food and health applications.

### STATE OF THE ART

Coffee has been one of the most cost-effective crops for many years. However, although productivity has continued to increase, the method used to process the fruit into grain has not changed for years and little attention has been paid to the use of the by-products produced when processing coffee. In coffee producing countries, the waste and by-products of the same constitute a serious source of pollution and environmental problems. For this reason, methods for using them as raw materials to produce animal feed, drinks, vinegar, biogas, caffeine, pectin, protein and fertilizer (http://www.ico.org/documents/edl967c.pdf) have been sought. As a product, coffee is of great commercial interest. Therefore, coffee by-products are widely available (Borrelli, R.C, Esposito, F, Napolitano, A, Ritieni, A and V Foglino. "Characterization of a New Potential Functional Ingredient: Coffee Silverskin. Journal of Agricultural and Food Chemistry, 2004, 52, 2: 1338-1343).

Many different by-products are obtained during the processing of the coffee fruit or cherry, right up until coffee powder is obtained to be consumed as a drink, namely pulp, coffee wastewater, parchment, mucilage, silverskin and spent grounds. The silverskin (or chaff) is the outer husk of the coffee bean and is the only coffee by-product produced as the coffee bean is roasted (Borrelli, R. C, Esposito, F, Napolitano, A, Ritieni, A and V Fogliano. "Characterization of a New Potential Functional Ingredient: Coffee Silverskin". Journal ofAgricultural and Food Chemistry, 2004, 52, 2: 1338-1343). It is currently used as a fuel or fertilizer (Saenger, M, Hartge, E. U, Werther, J, Ogada, T and Z Siagi. "Combustion of coffee husks". Renewable Energy, 2001, 23, 1: 103-121).

Coffee silverskin contains various phenolic compounds and other bioactive "phytochemical" food compounds, the properties of which present potential health benefits. Silverskin or coffee chaff has been proposed as a natural source of prebiotic ingredients, fibre and antioxidants (Borrelli, R.C. *et al.,* **2004,** as mentioned above). Studies carried out to date indicate that low concentrations of those phenolic compounds occurring naturally in the coffee bean, namely chlorogenic acid, are found in low concentrations in the silverskin. Indeed, the study carried out by Borrelli, R.C. et al mentioned above specifically states that 1.1 mg of phenolic compounds can be found in the silverskin per 100g of product, in the form of chlorogenic acid, when extracted in a ratio of 60:40 methanol/water. This article mentions that compounds derived from these phenolic compounds exist, which could contribute to the high antioxidant capacity attributed to this by-product produced in the coffee roasting industry. The content of the phenolic compounds and the antioxidant properties of the extracts are determined according to the extraction method used to prepare a sample prior to analysis thereof. Therefore, the antioxidant properties of the silverskin have been underestimated. Likewise, the direct use of the silverskin as a food preservative and/or as a source of dietary antioxidants has not previously been proposed. To date, no studies have been carried out on how easy to digest the silverskin is or the stability of its antioxidant properties during the digestion process by means of digestive enzymes.

Murthy, P.S and M.M Naidu. "Recovery of Phenolic Antioxidants and Functional Compounds from Coffee Industry By-Products". *Food and Bioprocess Technology,* **2010,** DOI 10.1007/s11947-010-0363-z, [online] describes the process followed to obtain an extract enriched with chlorogenic acid, which entails thermal treatment, pulverisation, enzyme treatment, chromatography fractionation and extraction using organic solvents.

A suitable alternative for managing by-products produced in the coffee industry, in Western countries, is by processing them using environmentally friendly technology, preferably without using organic solvents, which make it possible to obtain products with high added value. To the best of our knowledge, research carried out so far has not covered optimising the extraction of antioxidant compounds, specifically chlorogenic acid and caffeine, from the coffee silverskin, in order to later be used in foods, cosmetics and health products. Similarly, there is no evidence of previous research indicating that the coffee silverskin is a natural source of caffeine.

### DESCRIPTION OF THE INVENTION:

One initial aspect of the present invention relates to a product that comes from coffee silverskin, characterized in that it is (a) powdered coffee silverskin or (b) coffee silverskin extract.

In accordance with a preferred embodiment, the coffee silverskin used to obtain the product that is the object of the present invention can be a by-product generated during the roasting of Arabica, Robusta or mixed varieties of bean.

In aqueous extracts from silverskin, chlorogenic acid and caffeine have been identified, both of which are bioactive compounds found in coffee, to which significant and varied biological properties have been attributed. The extracts in the present invention may contain quantities of these compounds in the order of ppm (>200 mg/kg of silverskin) and high antioxidant capacity (equal to values > 0.85 g of chlorogenic / 100 g of silverskin). As such, in accordance with another preferred embodiment of the present invention, when the product that comes from the silverskin is a coffee silverskin extract, said product may comprise a minimum of 80 mg of chlorogenic acid and 985 mg of caffeine per 100 g of extract. Likewise, the extracts in the present invention may contain maximum values of 326 mg /100 g of chlorogenic acid extract and 4000 mg / 100 g of caffeine extract when the silverskin comes from the Arabica variety. These values may be in the order of twice this amount if the raw material used is silverskin that comes from the roasting of Robusta coffee beans.

Chlorogenic acid, in particular 5-caffeoylquinic acid, the products generated from these via the Maillard reaction during roasting and the antioxidant fibre, could be responsible for the antioxidant properties of the aqueous extracts. These compounds could prevent ageing, age-related pathological processes, and oxidative stress, and be effective at controlling body weight. Prior studies demonstrate that both green coffee extracts and coffee beverage may have this benefit, and these positive aspects are associated with the combined presence of the compounds detected in the extracts that are the object of the present invention.

A second aspect of the present invention is a method for obtaining coffee silverskin extract as disclosed in this patent application, characterized in that said method does not use organic solvents. Thus, the method of the present invention comprises a simple and environmentally-friendly extraction stage. In particular, the extraction may take place through enzymatic hydrolysis, cell breakage using mechanical methods at room temperature, hot water extraction, or extraction in subcritical and/or supercritical water conditions, thus enabling obtaining higher extraction yields and better characterisation of the silverskin, as well as possible new applications for the re-valorisation of this by-product.

In accordance with a preferred embodiment of this invention, the method for obtaining the coffee silverskin extract defined in the present patent application is characterised in that it comprises a water extraction stage.

Preferably, the method in the present invention does not comprise any prior grinding, pulverisation or other such stage for obtaining the powdered silverskin. As such, through the method that is the object of the present invention, the extract defined in the present application can be obtained from whole coffee silverskin, thereby saving time and energy and simplifying the method. Moreover, using powdered silverskin as a raw material could cause clumping during the extraction process, which could in turn give rise to less effective extraction.

Preferably, extraction may take place with water at a temperature of between 40 and 100 °C, although it is more preferable still for the extraction stage to take place with 2 volumes of water per gram of silverskin, at a temperature of roughly 100 °C for at least 10 min. More preferably still, without any prior grinding or other such process for obtaining the powdered silverskin. The studies carried out have shown that if a smaller amount of water is used, the silverskin clumps together and extraction is less effective. Likewise, a larger amount of water results in greater energy consumption in order to eliminate the solvent following extraction.

In accordance with another, additional preferred embodiment, this method for obtaining the coffee silverskin extract defined in the present patent application, which comprises a stage of extraction with water, is characterised in that the extraction stage may take place in extraction conditions with subcritical water.

Preferably, the subcritical water extraction stage as described above may take place at a temperature of between 50 and 200 °C and with enough constant pressure to keep the water in liquid state throughout the extraction. It is especially preferable to be able to constantly maintain the pressure between 1000 and 3000 psi.

In accordance with an especially preferred embodiment, the subcritical water extraction stage, as has been described in the present application, may take place at a temperature of approximately 50 °C and at a pressure of approximately 1500 psi, without a prior grinding, pulverisation or other such stage to obtain the powdered silverskin.

The presence of chlorogenic acids and caffeine in the sample extracts was analysed by means of HPLCD-MS and CZE-UV-Vis. The results indicate that the coffee silverskin has the potential to be used directly as a functional food ingredient and in the manufacture of anti-ageing cosmetics.

In accordance with another, additional preferred embodiment, the method for obtaining the coffee silverskin extract defined in the present patent application, which comprises a stage of extraction with water as defined in any one of the embodiments of the present invention, may comprise a prior grinding or similar such stage in order to obtain powdered silverskin.

For long-term storage, the extracts obtained according to the method described in the present patent application may be frozen, freeze-dried or atomised. The resulting powder may be stored out of the light in frozen form until being analysed. Assessment of the sample's antioxidant capacity while kept at room temperature shows that, under these conditions, they keep their antioxidant properties for at least 6 months.

A third aspect of the present invention relates to a foodstuff enriched with chlorogenic acid and caffeine, which comprises a product that comes from coffee silverskin as has been defined above in the present application, or a coffee silverskin extract obtained through the method that is the object of the present invention.

The total antioxidant capacity of the silverskin powder was determined, along with the soluble fraction obtained via *in vitro* gastrointestinal digestion of the same and of the compounds extracted with hot water (40-100°C) and in subcritical conditions (50-200°C, 1000 and 3000 psi). The soluble compounds derived from *in vitro* gastrointestinal digestion have antioxidant properties. As such, the dietary consumption of silverskin may strengthen the body's antioxidant defences. We know of no prior data attesting to the digestibility of silverskin and the consequent potential bioavailability of the antioxidant compounds that make it up. Silverskin powder could be used directly as a preservative just as spices are used.

A fourth aspect of the present invention relates to a cosmetic composition characterised in that it comprises a product that comes from coffee silverskin as has been defined above in the present application, or a coffee silverskin extract obtained through the method that is the object of the present invention and at least one suitable cosmetic excipient. This composition may be, for example, a lotion, tonic, emulsion, cream, ointment, powder (e.g. as a base for make-up or blush), or any other form common to the cosmetic industry.

Suitable cosmetic excipient means any excipient common to the cosmetic industry, e.g. extenders such as olive oil or allantoin, thickeners, fluidisers, aromas, agglutinatives, etc.

The coffee silverskin extract that is the object of the present invention can play various important roles in cosmetic compositions, such as preservative, aromatic additive, antioxidant and anti-ageing component. Because caffeine is another one of the fundamental compounds of the extract, and this compound is associated with an anti-cellulite effect, this is another property that could be attributed to the cosmetic product prepared by adding the extracts that are the object of the invention.

A fifth aspect of the present invention is the use of a product that comes from coffee silverskin as defined above or a coffee silverskin extract obtained through the method that is defined in the present patent application, such as an antioxidant and/or a natural preservative.

A sixth aspect of the present invention is the use of a product that comes from coffee silverskin as has been defined in this present application, of a coffee silverskin extract obtained through the method that is the object of the present invention or of a cosmetic composition as has been defined above, in order to present physiological ageing processes.

A seventh aspect of the present invention is the use of a product that comes from coffee silverskin as has been defined in this patent application, of a coffee silverskin extract obtained through the method that is the object of the present invention, or a foodstuff enriched with chlorogenic acid and caffeine as has been defined above, in order to prevent pathological processes related to ageing or oxidative stress.

An eighth aspect of the present invention is the use of a product that comes from coffee silverskin as defined in this patent application, of a coffee silverskin extract obtained through the method that is the object of the present invention, as a source of caffeine.

Preferably, the method for obtaining the coffee silverskin extract used as a source of caffeine comprises a stage of extraction with water, and is characterised in that the extraction stage may take place in subcritical water extraction conditions.

Preferably, the subcritical water extraction stage as described above with may take place at a temperature of between 50 and 200 °C and with enough constant pressure to keep the water in liquid state throughout the extraction. It is especially preferable to be able to constantly maintain the pressure between 1000 and 3000 psi.

In accordance with an especially preferred embodiment, the subcritical water extraction phase as has been described in the present application, in order to obtain an extract to use as a source of caffeine, may take place at a temperature of approximately 50°C and at a pressure of 1500 psi, without a prior grinding, pulverisation or other such stage to obtain the powdered silverskin.

### SUMMARY OF THE DRAWINGS:

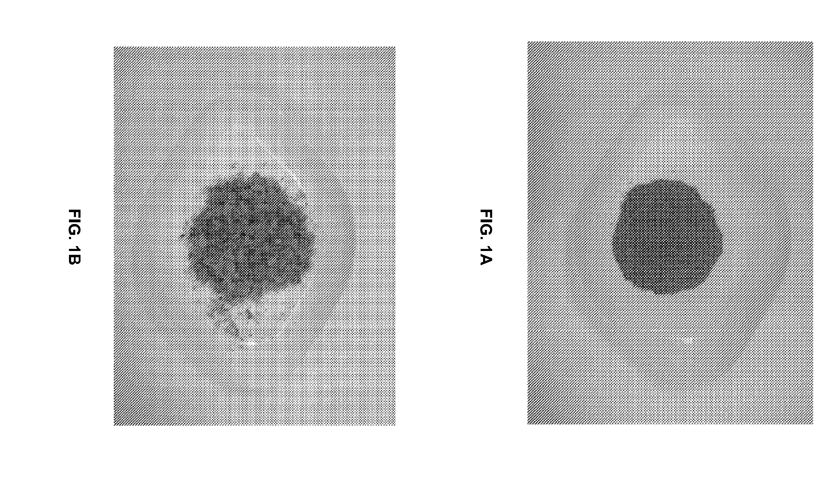

Figure 1a: Photograph of powdered silverskin.
Figure 1 b: Photograph of whole silverskin.
Figure 2: Freeze-dried powder from an aqueous silverskin extract.
Figure 3: Antioxidant capacity expressed as equivalents of chlorogenic in the silverskin samples and the products of their *in vitro gastrointestinal digestion.* The bars represent the mean of the assessment carried out in triplicate and the error bars denote the relative standard deviation of the measurements.
Figure 4: Electropherograms corresponding to the coffee silverskin extract extracted with hot water (A) and with subcritical water at 50°C (B). The initials CGA refer to the electrophoretic peaks with a UV-Vis spectrum with an agreement index of more than 80 % corresponding to a pure standard of chlorogenic acid (3-caffeoylquinic acid).

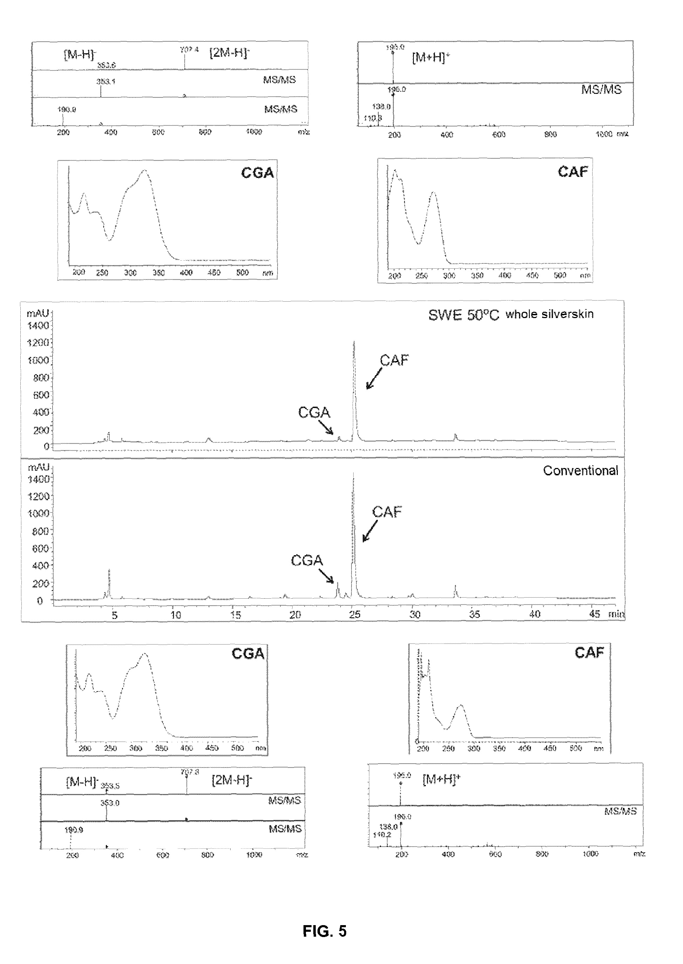

Figure 5: Identification of chlorogenic acid (CGA) and caffeine (caf) in aqueous extracts obtained through hot-water extraction at 100°C and in subcritical conditions at 50°C. The analysis has been carried out using HPLC with UV-Vis detection and mass spectrometry. A peak is identified with a retention time equal to that which is obtained by injecting the pure commercial standard of 3-caffeoylquinic acid. The chromatographic peak is the one with the greatest area of those showing similar spectral characteristics to the pure compound. Other minor peaks with a UV-Vis spectrum characteristic of CGA with area values lower than the quantification limit have not been taken into account when calculating the data shown in Table 2. The mass spectra confirm the identity of the compound that elutes as chlorogenic acid at this retention time. The spectra corresponding to the peaks detected in the extract obtained in subcritical conditions are shown in the top portion of the figure, whereas in the bottom portion those corresponding to hot-water extraction can be observed.

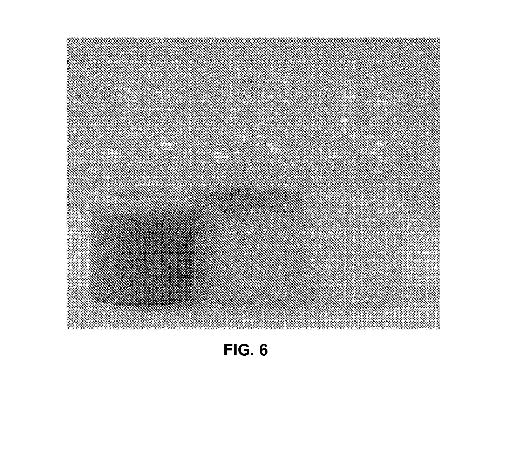

Figure 6: Aqueous silverskin extract based lotion (left), whole silverskin based lotion (centre), and basic lotion without added silverskin (right).

### ILLUSTRATIVE EXAMPLES OF THE INVENTION

### EXAMPLE 1: Obtaining the powdered silverskin

The silverskin powder is obtained by grinding the whole silverskin in a ball mill, using liquid nitrogen as a cooling system in order to avoid the loss of bioactive compounds such as chlorogenic acid, and running at 22 oscillations per second for 2 minutes.

### EXAMPLE 2: Antioxidant properties of the coffee silverskin and the in vitro gastrointestinal digestion thereof

The total antioxidant capacity of the powdered silverskin simple was determined by means of the direct ABTS method (Serpen, A, Capuano, E, Fogliano, V and V.A. Gokmen. "New Procedure to Measure the Antioxidant Activity of Insoluble Food Components". Journal of Agricultural and Food Chemistry, 2007, 55, 19: 7676-7681; Serpen, A, Gokmen, V, Pellegrini, N and V Fogliano. "Direct Measurement of the Total Antioxidant Capacity of Cereal Products". Journal of Cereal Science, 2008, 48, 816-820). The antioxidant capacity of the silverskin was also analysed by means of the GAR method recently proposed by Delgado-Andrade, C, Conde-Aguilera, J. A, Haro, A, de la Cueva, S. P, and J. A. Rufián-Henares. "A combined procedure to evaluate the global antioxidant response of bread". Journal of Cereal Science, 2010, 52: 239-246. This entailed analysing the antioxidant capacity of the digestive products obtained as a result of digestive enzyme activity, these enzymes occurring naturally in the human gastrointestinal tract, simulating the physiological conditions. This was determined in order to obtain information on the stability of the antioxidant compounds in the enzyme digestion process and the pH changes that occur during the digestion process. Following this method, we can predict that the antioxidant compounds do indeed reach the small intestine and are absorbed by cells in the gastrointestinal epithelium.

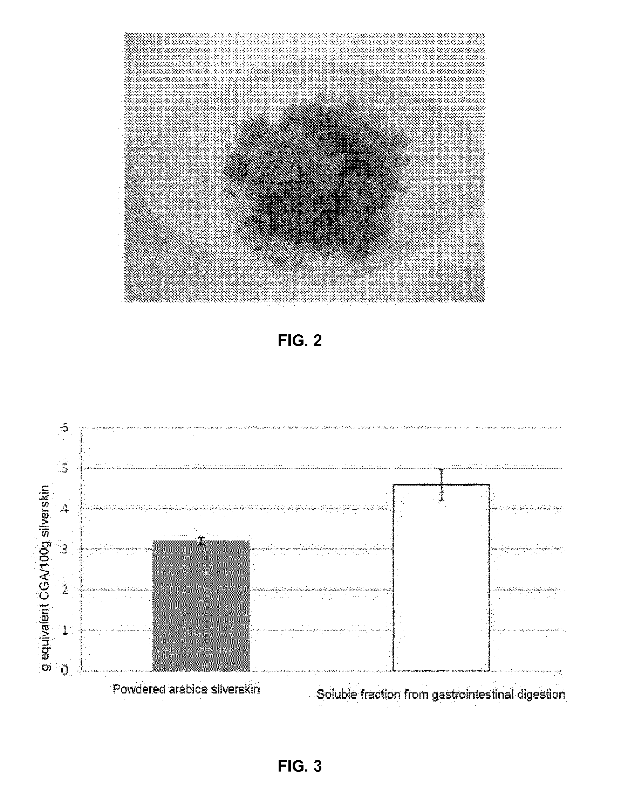

The results indicate that both the silverskin when intact and the digestive products thereof possess antioxidant capacities (Figure 3). The soluble digestive products may be absorbed via the intestinal epithelium and thus take effect on the organism. As a result, dietary consumption thereof may strengthen the body's antioxidant defences.

### EXAMPLE 3: Subcritical water extraction (SWE)

The extractions were carried out in an Accelerated Extraction system using ASE200 solvents marketed by Dionex, equipped with a solvent controller unit (Dionex, Sunnyvale, CA, USA).

The extractions were carried out in duplicate. All the extractions were carried out in an 11 ml capacity cell using 1 g of powdered coffee silverskin obtained from a coffee mixture consisting of Arabica coffee from various geographical origins (ground using a ball mill as indicated in example 1) or 500 mg when the silverskin is extracted without grinding. The data presented in the present document correspond to the extractions made at 50, 100, 150 and 200°C, maintaining a constant static extraction time (20 min). Likewise, the pressure was also kept constant at adequate values (1500 psi), in order to ensure the water remained in a liquid state throughout the entire extraction process, regardless of the temperature employed. The resulting extracts were frozen and the water eliminated by means of freeze-drying using a Labconco freeze-dryer (model 79480, Labconco Corporation, Missouri, USA). The dry extracts were kept at 4°C and protected from the light until they were analysed.

These extracts were also subjected to antioxidant analysis by means of the methods ABTS (Re, R, Pellegrini N, Proteggente, A, Pannala, A, Yang, M and C Rice-Evans. "Antioxidant Activity Applying an Improved ABTS Radical Cation Decolorization Assay". Free Radical Biology & Medicine, 1999, 26, 9/10:1231-1237), ORAC (Ou, B, Hampsch-Woodill, M and R.L. Prior. "Development and Validation of an Improved Oxygen Radical Absorbance Capacity Assay Using Fluorescein as the Fluorescent Probe". Journal of Agricultural and Food Chemistry, 2001, 49, 10: 4619-4626) and Folin (Schmidt, B. M, Erdman, J. W and M. A Lila. "Effects of Food Processing on Blueberry Antiproliferation and Antioxidant Activity". Journal of Food Science, 2005, 70,6: s389-s394). The composition thereof was analysed by means of HLPC-MS-UV-Vis and CZE-UV-Vis (Del Castillo, M. D, Ames, J. M and M. H Gordon. "Effect of Roasting on the Antioxidant Activity of Coffee Brews". Journal of Agricultural and Food Chemistry, 2002, 50 (13): 3698-3703).

### Extracts obtained by means of subcritical water extraction (SWE)

The extracts obtained by means of SWE are rich in antioxidants (see Table 1 below).

**Table 1: The results are expressed in equivalent grams of chlorogenic acid per 100 g of silverskin. The various letters denote statistically significant differences between samples in the sample column (p < 0.05).**

| **EXTRACT** | **ABTS** | **ORACFL** | **FOLIN** |
|---|---|---|---|
| **CF50** | 3.03 ± 0.17^{a} | 0.75 ± 0.08^{a,b} | 0.66 ± 0.06^{a,e,f} |
| **CF100** | 1.76 ± 0.02^{b} | 1.00 ± 0.04^{b} | 0.71 ± 0.02^{a,e,f} |
| **CF150** | 2.53 ± 0.13^{c} | 1.54 ± 0.15^{c} | 7.80 ± 0.79^{c} |
| **CF200** | 3.67 ± 0.26^{d} | 3.89 ± 0.36^{d} | 4.63 ± 1.59^{d} |
| **CaMo 50** | 1.32 ± 0.10^{e} | 0.72 ± 0.04^{a} | 0.28 ± 0.00^{e} |
| **CaMo 100** | 2.00 ± 0.17^{b} | 0.79 ± 0.05^{a,b} | 0.30 ± 0.02^{e} |
| **CaMo 150** | 2.97 ± 0.25^{a} | 1.39 ± 0.14^{c} | 0.64 ± 0.03^{a,e,f} |
| **CaMo 200** | 2.62 ± 0.01^{c} | 3.99 ± 0.44^{d} | 1.48 ± 0.04^{f} |

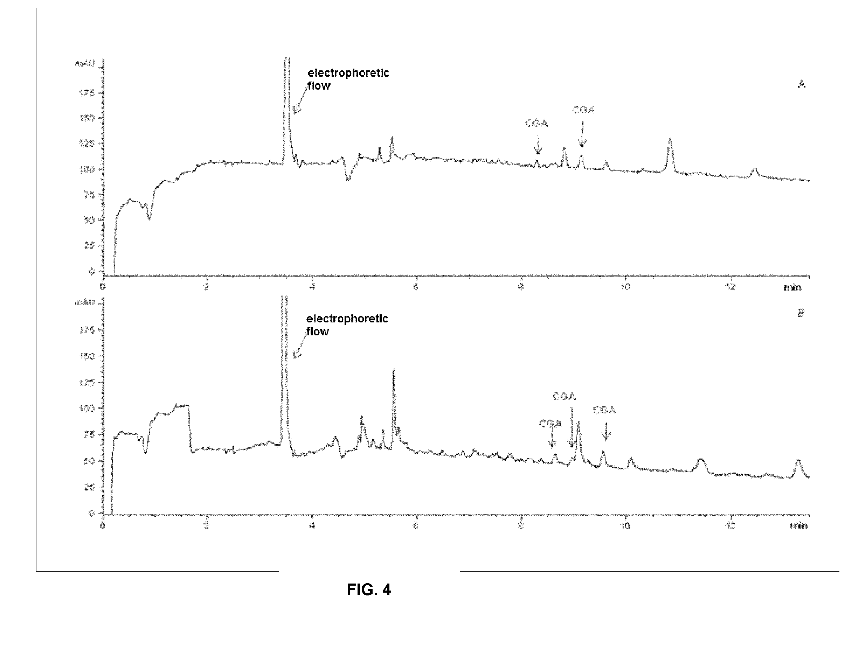

Therefore, they may potentially be used as a functional ingredient in foods, anti-aging compounds in cosmetics and as an antioxidant food supplement. The extracts contain chlorogenic acid and caffeine (Figures 4 and 5). The profile of phenolic compounds thereof is similar to that described for coffee beverage (Del Castillo, M. D, Ames, J. M and M. H Gordon. "Effect of Roasting on the Antioxidant Activity of Coffee Brews". Journal ofAgricultural and Food Chemistry, 2002, 50 (13): 3698-3703). Under these SWE conditions, amounts of up to 400 mg of chlorogenic acid may be extracted per Kg of silverskin and 4000 mg of caffeine per Kg of silverskin (see Table 2).

**Table 2. Chlorogenic acid and caffeine content in the aqueous extracts obtained both by means of simple hot water extraction at 100°C, i.e. conventional extraction, as well as in sub-critical conditions at temperatures of 50, 100, 150 and 200°C. The extracts under these conditions were obtained from the unprocessed raw material (whole silverskin), as well as from silverskin ground in the conditions described above.**

| **Extraction conditions** | **Chlorogenic Acid content** | **Caffeine content** |
|---|---|---|
| | **(mg/Kg silverskin)** | **(mg/Kg silverskin)** |
| *SWE - Whole silverskin* | | |
| 50 °C | 400.0 ± 25.4^{a} | 4765.5 ± 68.7^{a} |
| 100 °C | 289.9 ± 2.9^{b,c} | 4372.9 ± 57.3^{b} |
| 150 °C | 282.8 ± 19.2^{b,c} | 4855.1 ± 43.3^{a} |
| 200 °C | 272.6 ± 5.5^{c} | 3805.4 ± 21.3^{c} |

| *SWE - Ground silverskin* | | |
|---|---|---|
| 50 °C | 286.0 ± 2.8^{b,d} | 4508.2 ± 20.5^{b,d} |
| 100 °C | 200.6 ± 1.9^{d} | 4571.0 ± 33.3^{d} |
| 150 °C | 279.1 ± 15.2^{b,c} | 4517.2 ± 213.4^{d} |
| 200 °C | 295.5 ± 10.8^{b} | 4047.2 ± 36.8^{e} |

| *Conventional extraction* | | |
|---|---|---|
| 100 °C | 237.6 ± 0.5^{e} | 1473.0 ± 27.9^{f} |

| | | |
|---|---|---|
| Identical superscripts indicate results that are not statistically different (p > 0.05) | | |

The statistical analysis of the results for antioxidant properties, determined by using three standard models, indicate that the antioxidant power of the extracts depends, to a large extent, on the extraction conditions employed in each specific case (Table 1). We may furthermore conclude that extraction yields are better when unprocessed raw material is used to obtain the extract. The grinding step is not therefore necessary, and skipping this step results in significant savings, in terms of both time and energy costs.

### EXAMPLE 4: Hot water extraction

The extracts were obtained from powdered coffee silverskin produced as a by-product of Arabica coffee. The silverskin powder was obtained in a ball mill, using liquid nitrogen, operating at 22 oscillations per second for 2 minutes. The extracts were prepared in duplicate, using 250 g of raw material and 500 ml of water. The extraction was carried out at 100 °C for 10 minutes. The extracts thus obtained were spun and the supernatant portion collected, before being stored at temperatures of 5-8°C to await analysis.

The antioxidant capacity of the extracts obtained using the ABTS method (Re, R, Pellegrini N, Proteggente, A, Pannala, A, Yang, M and C Rice-Evans. "Antioxidant Activity Applying an Improved ABTS Radical Cation Decolorization Assay". Free Radical Biology & Medicine, 1999, 26, 9/10:1231-1237). The presence of chlorogenic acid in the extracts has been confirmed by means of HPLC with mass spectrometry detection and UV-Vis, and by means of CZE with UV-Vis detection (Del Castillo, M. D, Ames, J. M and M. H Gordon. "Effect of Roasting on the Antioxidant Activity of Coffee Brews". Journal ofAgricultural and Food Chemistry, 2002, 50 (13): 3698-3703).

### Extracts obtained by means of hot water extraction

The aqueous extract obtained from the coffee silverskin by means of hot water extraction had an equivalent antioxidant capacity of 0.85 g of chlorogenic acid per 100 g of silverskin. In order to facilitate storage of the extracts, they were freeze-dried and stored at room temperature. Experiments carried out to ascertain the stability of the powdered extract during storage at room temperature indicate that the antioxidant capacity thereof does not change for a period of over six months. The presence of chlorogenic acid and caffeine (Figures 4 and 5) was detected in these extracts. Table 2 shows the chlorogenic acid and caffeine content that may be extracted following this method.

### EXAMPLE 5: Cosmetic use

An emulsion was prepared by mixing light olive oil in 90% water in a household blender until the emulsion became homogenous. The emulsion prepared in this way had a pH value of 6.56 and a phenolic compound content in the order of 2.31 mg of Trolox per 100 ml of emulsion. Adding 0.4 % powdered silverskin extract (Figure 2) obtained using the extraction method described in example 4, followed by homogenisation, gave rise to a solution with a more suitable pH for applying to skin, of 5.44, which had a phenolic compound content in the order of 5 times greater than that detected in the base emulsion (10.98 mg of Trolox per 100 ml) and a high antioxidant capacity (117.42 mg of Trolox per 100 ml of lotion).

## Claims

1. Product that comes from coffee silverskin, **characterized in that** it is (a) powdered coffee silverskin or (b) coffee silverskin extract.

2. Product that comes from the coffee silverskin according to claim 1, **characterised in that** when it is a coffee silverskin extract, it comprises a minimum of 80 mg of chlorogenic acid and 985 mg of caffeine per 100 g of extract.

3. Method for obtaining a product that comes from coffee silverskin as defined in any of the claims 1 to 2, **characterized in that** when it is a coffee silverskin extract, said method does not use organic solvents.

4. Method according to claim 3, **characterised in that** it comprises a stage of extraction with water.

5. Method according to any of the claims 3 to 4, **characterized in that** the initial coffee silverskin has not undergone any prior grinding or other such process.

6. Method according to any of the claims 4 or 5, **characterised in that** it comprises an extraction stage with 2 volumes of water per gram of silverskin and said extraction takes place at roughly 100°C for at least 10 min.

7. Method according to any of the claims 4 or 5, **characterised in that** it comprises a subcritical water extraction stage.

8. Method according to claim 7, **characterised in that** the subcritical water extraction stage takes place between 50 and 200 °C and with enough constant pressure to keep the water in liquid state throughout the extraction.

9. Method according to claim 8, **characterized in that** the subcritical water extraction stage takes place at 50 °C, at an approximate pressure of 1500 psi and the initial coffee silverskin has not undergone any prior grinding or other such process.

10. Foodstuff enriched with chlorogenic acid and caffeine that comprises a product that comes from coffee silverskin as defined in any of the claims 1 or 2, or a coffee silverskin extract obtained through the method as defined in any of the claims 3 to 9.

11. Cosmetic composition **characterised in that** it comprises a product that comes from coffee silverskin as is defined in any of the claims 1 or 2 or a coffee silverskin extract obtained through the method as defined in any of the claims 3 to 9 and at least one suitable cosmetic excipient.

12. Use of a product that comes from coffee silverskin as defined in any of the claims 1 or 2 or a coffee silverskin extract obtained through the method as defined in any of the claims 3 to 9 as a natural antioxidant and/or natural preservative.

13. Use of a product that comes from coffee silverskin as is defined in any of the claims 1 or 2 of a coffee silverskin extract obtained through the method as defined in claims 3 to 9, or of a cosmetic composition as defined in claim 11, in order to prevent physiological ageing processes.

14. Use of a product that comes from coffee silverskin as defined in any of the claims 1 or 2, of a coffee silverskin extract obtained through the method as defined in any of the claims 3 to 9 or of a foodstuff enriched with chlorogenic acid and caffeine as defined in claim 10, in order to prevent pathological processes related to ageing or oxidative stress.

15. Use of a product that comes from coffee silverskin as defined in any of the claims 1 or 2, of a coffee silverskin extract obtained through the method as defined in any of the claims 3 to 9, as food preservative to prevent oxidative processes.

16. Use of a product that comes from coffee silverskin as defined in any of the claims 1 or 2, of a coffee silverskin extract obtained through the method as defined in any of the claims 3 to 9, as a source of caffeine.
